# EUROPEAN PATENT APPLICATION

(11) **EP 4 238 592 A1**
(43) Date of publication of application: **06.09.2023**
(21) Application number: 21886328.0
(22) Date of filing: 28.10.2021
(51) Int. Cl.: A61L 27/16, A61K 35/12, A61L 27/38, A61L 27/40, A61L 27/50, A61L 27/52, C12M 3/00

(54) **HYDROGEL FOR CELL EMBEDDING, IMMUNOISOLATION DEVICE, AND TRANSPLANT MATERIAL**

(30) Priority: 28.10.2020 JP 2020180920
(71) Applicant: Kuraray Co., Ltd., Kurashiki-shi, Okayama 710-0801 (JP)
(72) Inventor: KOBAYASHI, Goro, Tsukuba-shi, Ibaraki 305-0841 (JP); KAWAGOE, Masako, Osaka-shi, Osaka 530-8611 (JP); FUJITA, Akio, Tsukuba-shi, Ibaraki 305-0841 (JP); AYANO, Satoru, Tsukuba-shi, Ibaraki 305-0841 (JP)
(74) Representative: Müller-Boré & Partner Patentanwälte PartG mbB
(86) International application number: PCT/JP2021/039844
(87) International publication number: WO 2022/092211

(57) **Abstract**

An object of this invention is to simultaneously achieve, in long-term transplantation of a macroencapsulation immunoisolation device using a porous membrane or the like, improvement in the diffusion efficiency of physiologically active substances released from a material to be transplanted, improvement in the diffusion efficiency of nutrients to cells and the like, which are the material to be transplanted, and uniform and efficient dispersion and fixation of cells and the like encapsulated as the material to be transplanted in the macroencapsulation immunoisolation device, while maintaining an immunoisolation effect. The present invention provides a hydrogel for embedding a material to be transplanted, comprising a polymer crosslinked by visible light, and water.

## Description

### Technical Field

### Cross-Reference to Related Application

This application claims priority to Japanese Patent Application No. 2020-180920 filed on October 28, 2020 (the disclosure of which is incorporated herein by reference). The present invention relates to a hydrogel for embedding cells, an immunoisolation device, and a transplantation material.

### Background Art

Immunoisolation devices have been developed as a means for performing cell transplantation therapy without the need to administer an immunosuppressant. In particular, in transplantation of somatic cells derived from iPS cells, for which the risk of canceration is a concern, a decrease in the function of transplanted cells, or the like, macroencapsulation immunoisolation devices are considered an effective method in that the transplantation site can be identified and that the devices can be replaced.

Important functions required as macroencapsulation immunoisolation devices are the following: cells or cell clusters can be uniformly fixed in a dispersed state without aggregation; oxygen and/or nutritional components are allowed to easily permeate transplanted cells; desired physiologically active substances (cytokines, hormones, growth factors, etc.) that are released by cells and are required for a therapeutic effect can easily be released in response to the cells, and permeation of immunoresponsive cells and immune response factors can be prevented; and the transplanted devices are excellent in biocompatibility, and are less likely to adhere to surrounding tissue and less likely to induce inflammatory responses, such as granulation.

Many immunoisolation devices using porous membranes etc. have been studied, but have problems such as a decrease in permeability due to protein adsorption to porous membrane materials, a decrease in permeability due to fibrosis, and a decrease in permeability due to adhesion to surrounding tissue. The inventors have achieved fibrosis reduction by using a porous membrane made of an ethylene vinyl alcohol copolymer (EVOH), which has excellent biocompatibility.

For long-term engraftment of cells and the like, which are a graft, it is important to suppress the contact of immunoresponsive cells and immune response factors such as IgG with the graft, as well as to prevent the aggregation of cells and the like, which are a graft, (increased cell cluster) in the device and to shorten the diffusion distance from the recipient's body fluid and blood, which are sources of oxygen and nutrients to cells and the like, to the graft.

Internal necrosis of cell clusters is considered to be induced when the diameter exceeds 500 µm. In order to easily maintain engraftment of embedded cells to a recipient and release physiologically active substances, it is necessary to appropriately control the overall thickness of such a device. For this, the selection of a substrate for dispersion and fixation of cells and the like and the technique for fixation of cells and the like are extremely important.

Known methods for dispersion and fixation of cells and the like use fiber materials such as non-woven fabrics, woven fabrics, and meshes; hydrogels such as alginic acid and gelatin; or biomaterials such as collagen. However, when fiber materials are used, it is not easy to uniformly disperse cells and the like, and the above hydrogels and biomaterials are gradually biodegraded in the body over a long period of time. Thus, these materials are not considered to be optimal as materials for long-term stable retention of cells and the like.

To maintain the functionality of cells and the like, it is desirable to uniformly disperse and fix cells and the like, appropriately control the thickness of the fixation support layer, and shorten the material diffusion distance as much as possible. Thus, important components of immunoisolation devices are a hydrogel that is formed of a material ensuring safety without adversely affecting cells and the like, which are a graft, enables uniform dispersion and fixation of cells with ease of operation, and is irreversibly stable; and an immunoisolation membrane that has excellent long-term physical and chemical stability and at the same time maintains the functionality of cells and the like by controlling the permeability and diffusion distance.

### Citation List

### Patent Literature

PTL 1: JP2007-314736A
PTL 2: Japanese Journal of Polymer Science and Technology, vol 69, No. 10, pp. 555-566 (Oct. 2012)

### Summary of Invention

### Technical Problem

An object of the present invention is to simultaneously achieve, in long-term transplantation of a macroencapsulation immunoisolation device using a porous membrane or the like, improvement in the diffusion efficiency of physiologically active substances released from a material to be transplanted, improvement in the diffusion efficiency of nutrients to cells and the like, which are the material to be transplanted, and uniform and efficient dispersion and fixation of cells and the like encapsulated as the material to be transplanted in the macroencapsulation immunoisolation device, while maintaining an immunoisolation effect.

### Solution to Problem

As a solution, modified polyvinyl alcohol hydrogels (hereinafter "MPVAG"), which have good biocompatibility and easily undergo hydrogelation in the visible light region without adversely affecting cells, are used as a material for fixing cells (Patent Literature 1 and Patent Literature 2).

By adjusting the degree of crosslinking of the photocrosslinking agent of MPVAG, the concentration of MPVAG, or the like, it is possible to control the permeability of substances and suppress the permeation of immune response factors such as immunoresponsive cells and antibodies, without suppressing the permeation of desired physiologically active substances.

As a method for uniformly dispersing and fixing cells and the like, a cell dispersion obtainable by suspending and dispersing cells and the like in a sol solution of MPVAG can be seeded on, for example, a microdimple sheet having dimples with a diameter of 50 to 500 µm or a micro-device having slits with a width of 50 to 500 µm, followed by photocrosslinking with visible light of 360 nm or more, thereby preparing a hydrogel sheet without internal necrosis of cell clusters while maintaining a certain distance without aggregation of cells and the like. Such microdimple sheets and hydrogel sheets can be used as transplantation materials as they are, or can be introduced into a macroencapsulation immunoisolation device covered with an immunoisolation membrane to prepare transplantation materials.

The present invention provides the following hydrogel for embedding a material to be transplanted and transplantation material.
[1] A hydrogel for embedding a material to be transplanted, comprising a polymer crosslinked by visible light, and water.
[2] The hydrogel for embedding a material to be transplanted according to [1], wherein the polymer is a polyvinyl alcohol-based polymer.
[3] An immunoisolation device comprising the hydrogel for embedding a material to be transplanted according to [1] or [2].
[4] A transplantation material comprising a material to be transplanted that is embedded in the hydrogel according to [1] or [2] .
[5] A transplantation material comprising a material to be transplanted that is embedded in a hydrogel in an embedding chamber covered with an immunoisolation membrane of a macroencapsulation immunoisolation device, the hydrogel comprising a polymer crosslinked by visible light, and water.
[6] The transplantation material according to [5], wherein the immunoisolation membrane comprises a hydrogel.
[7] The transplantation material according to [5] or [6], wherein the visible light has a wavelength of 360 nm or more.
[8] The transplantation material according to any one of [5] to [7], wherein the immunoisolation membrane is capable of removing 50% or more of immunoresponsive cells and immune system humoral factors.
[9] The transplantation material according to any one of [5] to [8], wherein the immunoisolation membrane has insulin and glucose permeabilities of 95% or more and an immune system humoral factor permeability of 10% or less, without permeation of immunoresponsive cells.
[10] The transplantation material according to any one of [5] to [9], wherein the immunoisolation membrane has a thickness of 20 µm to 500 µm, the material to be transplanted is a plurality of cells or cell clusters, and the plurality of cells or cell clusters embedded in the hydrogel are arranged at an interval of 10 µm or more and 500 µm or less without contacting each other.

### Advantageous Effects of Invention

The present invention provides a hydrogel for embedding a material to be transplanted that can encapsulate a material to be transplanted such as cells and cell clusters and restrict the movement and spacing thereof. This hydrogel not only protects cells and/or cell clusters by spacing adjacent cells and/or cell clusters apart from each other, but also prevents contact between immunoresponsive cells and the cells or cell clusters, thus preventing attack by immunoresponsive cells on the transplanted cells or cell clusters. Further, by introducing a material to be transplanted that is embedded in the hydrogel into an embedding chamber enclosed by an immunoisolation membrane, the effects of immunoresponsive cells and immune system humoral factors can be eliminated.

The hydrogel for embedding a material to be transplanted of the present invention may be used alone as an immunoisolation device, or may be placed in an embedding chamber enclosed by an immunoisolation membrane and used as an immunoisolation device.

### Brief Description of Drawings

Fig. 1 shows a perspective view of a bag-shaped immunoisolation device.
Fig. 2 shows a cross-sectional view of a tubular immunoisolation device.
Fig. 3 shows a cross-sectional view of a three-layer immunoisolation membrane having a joining part between the porous membrane and the hydrogel.
Fig. 4 shows an example of a mold for the production of a hydrogel for an immunoisolation membrane using microfabrication technology to uniformly disperse and fix cells and the like.
Fig. 5 shows a concept view of a hydrogel sheet comprising cells and the like, which is produced using the mold.
Fig. 6 shows an entire view of the mold.
Fig. 7 shows a micrograph of a hydrogel sheet taken in Example 2.

### Description of Embodiments

The hydrogel for embedding a material to be transplanted of present invention comprises a polymer crosslinked by visible light, and water. The visible light may be, for example, light with a wavelength of 360 to 830 nm, or 360 to 800 nm, and also include, for example, light with a wavelength of 500 to 600 nm. The hydrogel for embedding a material to be transplanted can be prepared by irradiating a composition (e.g., a suspension) containing a hydrosol and a material to be transplanted with visible light. The temperature of the hydrosol during visible light irradiation is not limited, and is, for example, about 4 to 40°C. The irradiation time is also not limited, and is, for example, about 10 seconds to 15 minutes.

The thickness of the transplantation material in which a material to be transplanted is embedded in the hydrogel is not particularly limited, and is preferably 100 µm to 500 pm, and more preferably 150 um to 300 µm.

The hydrogel for embedding has a tensile strength at break of preferably 0.01 to 10 MPa, and more preferably 0.03 to 1 MPa.

The polymer crosslinked by visible light can be obtainable by crosslinking a polymer that can be crosslinked by visible light, by visible light. The polymer that can be crosslinked by visible light has a hydrophilic skeleton capable of forming a hydrogel in the presence of water and a polymerizable group that is crosslinkable by visible light. The polymer that can be crosslinked by visible light is preferably a polyvinyl alcohol-based polymer. The polyvinyl alcohol-based polymer has an ethylenically unsaturated group.

The polyvinyl alcohol-based polymer used in the present invention is not particularly limited as long as it has an ethylenically unsaturated group and contains more than 50 mol% of a structural unit derived from vinyl alcohol. The polyvinyl alcohol-based polymer may contain a structural unit derived from vinyl ester. The total amount of the structural unit derived from vinyl alcohol and the structural unit derived from vinyl ester is preferably 80 mol% or more, more preferably 90 mol% or more, and even more preferably 95 mol% or more, based on all the structural units constituting the polyvinyl alcohol-based polymer.

The ethylenically unsaturated group is not particularly limited and can be freely selected. The ethylenically unsaturated group is preferably a group that can form a crosslink between polyvinyl alcohol-based polymer chains by visible light. As the ethylenically unsaturated group, it is more preferable to use a radically polymerizable group. Examples include a vinyl group, a (meth)acryloyloxy group, a (meth)acryloylamino group, a vinylphenyl group, a cyclohexenyl group, a cyclopentenyl group, a norbornenyl group, and like cyclic unsaturated hydrocarbon groups, and derivatives thereof. These ethylenically unsaturated groups may be present at either side chains or termini of vinyl alcohol-based polymer chains.

The "vinyl group" in the present specification includes not only an ethenyl group, but also chain unsaturated hydrocarbon groups such as an allyl group and an alkenyl group, vinyloxycarbonyl, and the like.

Among the radically polymerizable groups, at least one member selected from the group consisting of a vinyl group, a (meth)acryloyloxy group, a (meth)acryloylamino group, a vinylphenyl group, a norbornenyl group, and derivatives thereof is preferred from the viewpoint of improving the mechanical strength of hydrogel particles. From the viewpoint of reactivity, a functional group with a terminal unsaturated carbon bond is preferred, and a (meth)acryloyloxy group is more preferred.

The range of the average polymerization degree of the polyvinyl alcohol-based polymer is not limited, and is, for example, 300 to 10,000, preferably 450 to 5,000, more preferably 500 to 3,000, and most preferably 500 to 2,500. The vinyl alcohol-based polymer may be a mixture of two or more vinyl alcohol-based polymers having different average polymerization degrees. The average polymerization degree is preferably 300 or more from the viewpoint of suppressing embrittlement of the hydrogel of the present invention. The average polymerization degree is also preferably 10,000 or less, more preferably 5,000 or less, and even more preferably 3,000 or less, from the viewpoint of keeping the viscosity of an aqueous vinyl alcohol-based polymer solution within a range that is easy to handle.

The average polymerization degree of the vinyl alcohol-based polymer in the present specification refers to the average polymerization degree measured according to JIS K 6726:1994. Specifically, since the polymerization degree of the vinyl alcohol-based polymer can be considered to be the same as the polymerization degree of the raw material PVA described below, the average polymerization degree can be determined from the intrinsic viscosity that is measured in water at 30°C after the raw material PVA has been purified.

### Method for Producing Vinyl Alcohol-based Polymer

Examples of the method for producing the vinyl alcohol-based polymer used in the present invention, i.e., a polyvinyl alcohol-based polymer having an ethylenically unsaturated group, include a method of introducing an ethylenically unsaturated group via a side chain, a terminal functional group, or the like of polyvinyl alcohol used as a raw material (which, hereinafter, may be referred to as the "raw material PVA"); and a method of introducing an ethylenically unsaturated group by copolymerizing a vinyl ester-based monomer and a polymerizable monomer that is not a vinyl ester-based monomer and has a reactive substituent other than a hydroxyl group in the process of producing the raw material PVA, and then reacting the reactive substituent in the resulting copolymer with a compound containing an ethylenically unsaturated group.

The raw material PVA can be produced by saponification of a polyvinyl ester obtainable by polymerizing a vinyl ester-based monomer, and converting the ester group in the polyvinyl ester to a hydroxyl group.

Examples of the vinyl ester-based monomer include vinyl formate, vinyl acetate, vinyl propionate, vinyl n-butyrate, vinyl isobutyrate, vinyl pivalate, vinyl versatate, vinyl caproate, vinyl caprylate, vinyl caprate, vinyl laurate, vinyl myristate, vinyl palmitate, vinyl stearate, vinyl oleate, and like aliphatic vinyl esters; vinyl benzoate, and like aromatic vinyl esters. These vinyl ester-based monomers may be used alone or in a combination of two or more.

Among the vinyl ester-based monomers, aliphatic vinyl esters are preferred, and vinyl acetate is more preferred in view of production cost. In other words, the polyvinyl ester is preferably polyvinyl acetate obtainable by polymerization of vinyl acetate.

The polyvinyl ester may contain a structural unit derived from other monomers than vinyl ester-based monomers, as required, to the extent that the effect of the present invention is not impaired. Examples of other monomers include α-olefins, such as ethylene, propylene, n-butene, and isobutylene; acrylic acid or salts thereof; alkyl acrylates such as methyl acrylate, ethyl acrylate, n-propyl acrylate, i-propyl acrylate, n-butyl acrylate, i-butyl acrylate, t-butyl acrylate, 2-ethylhexyl acrylate, dodecyl acrylate, and octadecyl acrylate; methacrylic acid or salts thereof; alkyl methacrylates such as methyl methacrylate, ethyl methacrylate, n-propyl methacrylate, i-propyl methacrylate, n-butyl methacrylate, i-butyl methacrylate, t-butyl methacrylate, 2-ethylhexyl methacrylate, dodecyl methacrylate, and octadecyl methacrylate; acrylamide derivatives such as acrylamide, N-methylacrylamide, N-ethylacrylamide, N,N-dimethylacrylamide, diacetoneacrylamide, acrylamidopropane sulfonic acid or salts thereof, acrylamidopropyl dimethylamine or salts or quaternary salts thereof, N-methylolacrylamide or derivatives thereof; methacrylamide derivatives such as methacrylamide, N-methylmethacrylamide, N-ethylmethacrylamide, methacrylamidopropanesulfonic acid or salts thereof, methacrylamidopropyl dimethylamine or salts or quaternary salts thereof, N-methylolmethacrylamide or derivatives thereof; N-vinylamide derivatives such as N-vinylformamide and N-vinylacetamide; vinyl ethers such as methyl vinyl ether, ethyl vinyl ether, n-propyl vinyl ether, i-propyl vinyl ether, n-butyl vinyl ether, i-butyl vinyl ether, t-butyl vinyl ether, dodecyl vinyl ether, and stearyl vinyl ether; nitriles such as acrylonitrile and methacrylonitrile; vinyl halides such as vinyl chloride and vinyl fluoride; vinylidene halides such as vinylidene chloride and vinylidene fluoride; allyl compounds such as allyl acetate and allyl chloride; maleic acid or salts thereof, ester or acid anhydride; vinyl silyl compounds such as vinyl trimethoxysilane; isopropenyl acetate; etc. These can be used alone or in a combination of two or more.

When the polyvinyl ester contains a structural unit derived from another monomer, the content of the structural unit derived from the other monomer is preferably 20 mol% or less, more preferably 10 mol% or less, and even more preferably 5 mol% or less, based on all the structural units constituting the polyvinyl ester.

The method of saponifying the polyvinyl ester is not particularly limited, and conventionally used methods can be used. For example, an alcoholysis method or a hydrolysis method using an alkali catalyst or an acid catalyst can be used. In particular, an saponification reaction using methanol as a solvent and a caustic soda (NaOH) catalyst is simple and preferred.

The average polymerization degree of the raw material PVA in the present specification is the average polymerization degree measured according to JIS K 6726:1994, as described above. Specifically, the average polymerization degree can be determined from the intrinsic viscosity that is measured in water at 30°C after the raw material PVA has been saponified and purified.

The degree of saponification of the raw material PVA is preferably 50 mol% or more, more preferably 60 mol% or more, and even more preferably 65 mol% or more, from the viewpoint of improving the water solubility of the raw material PVA.

From the viewpoint of inhibiting an uncured gel solution described below from having high viscosity, and improving the storage stability of the uncured gel solution, the degree of saponification of the raw material PVA is preferably 99 mol% or less.

In the present specification, the saponification degree of the raw material PVA means the ratio (mol%) of the number of moles of the vinyl alcohol unit to the total number of moles of the structural unit (e.g. vinyl acetate unit) that can be converted to a vinyl alcohol unit by saponification in the raw material PVA and a vinyl alcohol unit. The saponification degree of the raw material PVA can be measured according to JIS K 6726:1994.

The 4 mass% viscosity at 20°C of the raw material PVA is preferably 0.5 to 100 mPa·s, more preferably 1 to 80 mPa·s, and even more preferably 2 to 60 mPa·s. When the viscosity is within the above ranges, the hydrogel can be more easily produced, and the strength of the hydrogel can be enhanced.

In the present specification, the viscosity refers to the viscosity of an aqueous solution containing 4 mass% of the raw material PVA at a temperature of 20°C measured using a B-type viscometer (rotation rate: 12 rpm) according to the rotational viscometer method of JIS K 6726:1994.

Introduction of an ethylenically unsaturated group into the raw material PVA is preferably performed via a side chain, a terminal functional group, or the like of the raw material PVA. More preferably, a compound containing an ethylenically unsaturated group (which, hereinafter, may be referred to as an "ethylenically unsaturated group-containing compound") is reacted with a hydroxyl group of a side chain of the raw material PVA.

Examples of the ethylenically unsaturated group-containing compound to be reacted with a hydroxyl group of a side chain of the raw material PVA include (meth)acrylic acid or derivatives thereof, such as (meth)acrylic acid, (meth)acrylic anhydride, (meth)acrylic acid halide, and (meth)acrylic acid ester. A (meth)acryloyl group can be introduced by subjecting these compounds to an esterification or transesterification reaction in the presence of a base.

Examples of the ethylenically unsaturated group-containing compound to be reacted with a hydroxyl group of a side chain of the raw material PVA also include compounds containing an ethylenically unsaturated group and a glycidyl group in the molecule, such as glycidyl (meth)acrylate and allyl glycidyl ether. A (meth)acryloyl group and/or an allyl group can be introduced into the raw material PVA by subjecting these compounds to an etherification reaction in the presence of a base.

Further, examples of the ethylenically unsaturated group-containing compound to be reacted with a 1,3-diol group of the raw material PVA include compounds containing an ethylenically unsaturated group and an aldehyde group in the molecule, such as acrylaldehyde (acrolein), methacrylaldehyde (methacrolein), 5-norbornene-2-carboxyaldehyde, 7-octenal, 3-vinylbenzaldehyde, and 4-vinylbenzaldehyde. An ethylenically unsaturated group can be introduced into the raw material PVA by subjecting these compounds to an acetalization reaction in the presence of an acid catalyst. More specifically, for example, 5-norbornene-2-carboxyaldehyde, 3-vinylbenzaldehyde, or 4-vinylbenzaldehyde may be subjected to an acetalization reaction to introduce a norbornenyl group or a vinylphenyl group into the raw material PVA. Moreover, N-(2,2-dimethoxyethyl)(meth)acrylamide or the like may be reacted to introduce a (meth)acryloylamino group into the raw material PVA.

In addition to the reactions described above, other methods can be used to introduce an ethylenically unsaturated group into the raw material PVA, and two or more reactions may be used in combination.

Another method for introducing an ethylenically unsaturated group is, for example, a method of copolymerizing a vinyl ester-based monomer with a polymerizable monomer that is not a vinyl ester-based monomer and has a reactive substituent other than a hydroxyl group in the process of producing the raw material PVA, saponifying the resulting copolymer to give copolymerized, modified polyvinyl alcohol (which, hereinafter, may be referred to as "copolymerized, modified PVA"), and then reacting the reactive substituent, such as the carboxy group present in the copolymerized, modified PVA or the amino group present in the copolymerized, modified PVA, with an ethylenically unsaturated group-containing compound. The copolymerized, modified PVA containing a carboxy group may be referred to as "carboxylic acid-modified PVA," and the copolymer containing an amino group may be referred to as "amino-modified PVA."

Examples of monomers that can be used to form carboxylic acid-modified PVA include α,β-unsaturated carboxylic acids such as (meth)acrylic acid, maleic acid, fumaric acid, and itaconic acid; alkyl (meth)acrylates such as methyl (meth)acrylate and ethyl (meth)acrylate; α,β-unsaturated carboxylic acid anhydrides and derivatives thereof such as maleic anhydride and itaconic anhydride; and the like. For carboxylic acid-modified PVA, for example, a vinyl ester-based monomer may be copolymerized with an α,β-unsaturated carboxylic acid anhydride or a derivative thereof, the resulting copolymer may be saponified, and then, for example, glycidyl methacrylate may be reacted with the introduced carboxy group under acid conditions to form an ester bond, thereby introducing a methacryloyl group.

For amino-modified PVA, a vinyl ester-based monomer may be copolymerized with N-vinylformamide or the like, the resulting copolymer may be saponified, and then, an amidation reaction of, for example, acrylic anhydride with the introduced amino group may be performed in the presence of a base, thereby introducing an acryloylamino group. A vinyloxycarbonyl group may be introduced by performing an amidation reaction of, for example, divinyl adipate with the amino group of the amino-modified PVA. In addition to the reactions described above, other methods can be used to introduce an ethylenically unsaturated group via a copolymerized, modified PVA, and two or more reactions may be used in combination.

From the viewpoint of ease of production, the polyvinyl alcohol-based polymer having an ethylenically unsaturated group is preferably a polyvinyl alcohol-based polymer having an ethylenically unsaturated group introduced via a hydroxyl group of a side chain of the raw material PVA, such as a 1,3-diol group, and is more preferably a vinyl alcohol-based polymer obtainable by performing an esterification or transesterification reaction of (meth)acrylic acid or a derivative thereof with a hydroxyl group of a side chain of the raw material PVA or a polyvinyl alcohol-based polymer obtainable by performing a acetalization reaction of a compound containing an ethylenically unsaturated group and an aldehyde group in the molecule with a 1,3-diol group of the raw material PVA.

### Ethylenically Unsaturated Group Introduction Rate

The ethylenically unsaturated group introduction rate is preferably 10 mol% or less, more preferably 5 mol% or less, and even more preferably 3 mol% or less in all the structural units constituting the vinyl alcohol-based polymer, from the viewpoint of suppressing embrittlement of hydrogel particles. From the viewpoints of promoting the crosslinking reaction, rapidly forming hydrogel particles, and improving the elastic modulus of the resulting hydrogel particles, the ethylenically unsaturated group introduction rate is preferably 0.01 mol% or more, more preferably 0.1 mol% or more, even more preferably 0.5 mol% or more. The ethylenically unsaturated group introduction rate is preferably within the range of 0.01 to 10 mol%, more preferably 0.1 to 5 mol%, and even more preferably 0.5 to 3 mol%.

Examples of other polymers that can be used in combination with the polyvinyl alcohol-based polymer include polyvinyl alcohol, collagen, chitosan, carboxymethyl cellulose, pullulan, poly(2-hydroxyethyl acrylate), poly(2-hydroxyethyl methacrylate), poly(N-isopropylacrylamide), polyacrylic acid, polymethacrylic acid, cellulose derivatives, polyvinylpyrrolidone, and copolymers obtained using at least one of the monomers that constitute these polymers.

Examples of the material to be transplanted include cells, cell clusters, grafts, and the like. The material to be transplanted is preferably a plurality of cells or cell clusters. The size of the material to be transplanted such as a cell cluster is preferably 500 µm or less in order to sufficiently supply oxygen and/or nutrients.

When the material to be transplanted that is embedded in the hydrogel for embedding is transplanted in a living body alone, a hydrosol for embedding that contains the material to be transplanted such as cells may be placed in a mold and irradiated with visible light to prepare a hydrogel-embedded material with necessary strength and permeability, and the material may be transplanted. When a macroencapsulation immunoisolation device is used in place of a mold, a hydrosol for embedding that contains the material to be transplanted may be placed in an embedding chamber enclosed by an immunoisolation membrane with a syringe or the like, visible light may be applied from outside to inside the immunoisolation membrane to convert the hydrosol to a hydrogel, and the device may be transplanted in a living body.

The preferred immunoisolation membrane comprises at least one member selected from the group consisting of fiber structures, porous membranes, and hydrogels. The thickness of the immunoisolation membrane is not particularly limited, and is preferably 20 µm or more and 500 µm or less.

The "fiber structure" is, for example, a non-woven fabric, a woven fabric, or a knitted fabric, and is preferably a non-woven fabric. Examples of materials of the fiber structure include gelatin, collagen, chitin, chitosan, fibronectin, dextran, cellulose, polyethylene (PE), polypropylene (PP), polyurethane, polyamide, polyester, polyvinyl alcohol (PVA), polylactic acid, polyglycolic acid, polylactic acid-polyglycolic acid copolymers, polyvinyl alcohol, polycaprolactone, polyglycerol sebacate, polyhydroxyalkanoic acid, polybutylene succinate, polymethylene carbonate, cellulose diacetate, cellulose triacetate, methylcellulose, propylcellulose, benzyl cellulose, carboxymethylcellulose, fibroin, silk, and the like. The fiber structure layer may be composed of one fiber structure layer, or two or more fiber structure layers may be laminated to form a single fiber structure layer. When the immunoisolation device of the present invention comprises two or more fiber structure layers, the fiber structure layers may be directly laminated, or a porous membrane layer or a hydrogel layer may be interposed between two fiber structure layers.

The thickness of the fiber structure layer is not particularly limited, and is preferably 200 µm or less, and more preferably 10 µm to 100 µm.

Examples of hydrosols for producing the "hydrogel" include sols that gel in the presence of a metal ion to form hydrogels; sols that gel in response to temperature to form hydrogels; sols that gel in response to pH to form hydrogels; sols that gel in response to light to form hydrogels; and the like. Metal ions and pH are examples of chemical action. To gel these hydrosols, an operation such as bringing a metal ion into contact with a hydrosol, adjusting the temperature to gelling conditions, adjusting the pH to gelling conditions, applying light under gelling conditions, or providing a magnetic field under gelling conditions may be performed depending on the characteristics of the gel used.

Examples of hydrogels obtainable by gelling in the presence of a metal ion include alginate gels obtainable by gelling in the presence of a divalent or trivalent metal ion, preferably an alkaline earth metal ion, such as a calcium ion or a magnesium ion; carrageenan gels obtainable by gelling in the presence of a calcium ion and/or a potassium ion; acrylic acid-based synthesis gels obtainable by gelling in the presence of a sodium ion; and the like.

Examples of temperature-responsive hydrogels include a temperature-responsive hydrogel obtainable by crosslinking poly(N-isopropylacrylamide) with polyethylene glycol (trade name: Mebiol Gel), methylcellulose, hydroxypropyl cellulose, a copolymer of lactic acid and ethylene glycol, a triblock copolymer of polyethylene glycol and polypropylene oxide (trade name: Pluronic (registered trademark), Poloxamer), agarose, polyvinyl alcohol, and the like.

Examples of pH-responsive hydrogels include alginate gels, chitosan gels, carboxymethyl cellulose gels, acrylic acid-based synthesis gels, and the like.

Examples of photoresponsive hydrogels include a synthesis gel in which azobenzene and cyclodextrin are combined in the skeleton, a gel composed of a supramolecule having fumaric acid amide as a spacer, a gel obtainable by crosslinking or bonding via a nitrobenzyl group, and the like.

The thickness of the hydrogel layer is not particularly limited, and is preferably 10 µm to 300 µm, more preferably 20 µm to 200 pm, and even more preferably 30 to 150 µm.

The hydrogel layer may be composed of one hydrogel layer, or two or more hydrogel layers may be laminated to form a single hydrogel layer. When the immunoisolation device of the present invention comprises two or more hydrogel layers, the hydrogel layers may be directly laminated, or a porous membrane layer or a fiber structure layer may be interposed between the two hydrogel layers.

The hydrogel can adjust the permeabilities of, for example, nutritional substances such as glucose, physiologically active substances such as insulin, and immune system humoral factors, the strength, etc. by crosslinking. The gel concentration of the hydrogel is not limited, and is preferably 2 mass% or more, and more preferably 4 mass% or more, from the viewpoint of gel strength. The upper limit of the gel concentration of the hydrogel is also not limited, and is preferably 12 mass% or less, and more preferably 8 mass% or less, from the viewpoint of viscosity that is easy to handle.

The "porous membrane" is a membrane with multiple pores. Whether a membrane is a porous membrane can be confirmed by a scanning electron microscope (SEM) image or a transmission electron microscope (TEM) image of the cross section of the membrane, or a model particle removal rate (latex etc.).

The thickness of the porous membrane is not particularly limited, and is preferably 500 um or less, more preferably 50 um or less, and even more preferably 20 µm or less.

The average pore size of the porous membrane is not particularly limited, and is preferably 0.001 um to 10 um, and more preferably 0.01 um to 3 µm.

The maximum pore size of the porous membrane is not particularly limited, and is preferably 0.01 um to 10 um, and more preferably 0.01 um to 3 um. When the porous membrane has a maximum pore size within the above ranges, the porous membrane can suppress the entry of immunoresponsive cells into the embedding chamber and achieves sufficient permeation of nutritional substances such as amino acids, vitamins, inorganic salts, and carbon sources(such as glucose); oxygen; carbon dioxide; and physiologically active substances such as cytokines, hormones, and insulins. The average pore size or the maximum pore size can be determined from an SEM image or a TEM image.

It is preferred that the porous membrane comprises a polymer and is substantially composed of the polymer. The polymer forming the porous membrane is preferably biocompatible.

The polymer may be, for example, a thermoplastic or thermosetting polymer. The polymer may be biocompatible. Specific examples of polymers include ethylene-vinyl alcohol copolymers, polysulfones, cellulose acetate and like cellulose acylate, nitrocellulose, sulfonated polysulfones, polyethersulfone, polyacrylonitrile, styrene-acrylonitrile copolymers, styrenebutadiene copolymers, saponified products of ethylene-vinyl acetate copolymers, polyvinyl alcohol, polycarbonates, organosiloxane-polycarbonate copolymers, polyester carbonate, organopolysiloxane, polyphenylene oxide, polyamides, polyimides, polyamideimides, polybenzimidazoles, polytetrafluoroethylene (PTFE), and the like. These may be homopolymers, copolymers, polymer blends, polymer alloys, etc. in terms of, for example, solubility, optical properties, electrical properties, strength, and elasticity. Hydrophilic polymers such as polyvinylpyrrolidone, hydroxypropyl cellulose, and hydroxyethyl cellulose may be contained as polymers forming the porous membrane. The biocompatibility can be improved by combining hydrophilic and hydrophobic polymers.

The porous membrane is preferably a membrane formed from one composition as a single layer and preferably does not have a multilayer, laminated structure.

Specifically, a method of forming a membrane using a phase separation reaction of a polymer can be used. For example, a solution of a polymer forming the porous membrane is applied to a substrate, such as glass, and exposed to a poor solvent in which the polymer is insoluble or a low-temperature environment to precipitate and solidify the polymer, thereby forming a porous membrane on the substrate.

The porous membrane layer may be composed of one porous membrane layer, or two or more porous membrane layers may be laminated to form a single porous membrane layer. When the immunoisolation device of the present invention comprises two or more porous membrane layers, the porous membrane layers may be directly laminated, or a hydrogel layer or a fiber structure layer may be interposed between two porous membrane layers.

The amount of glucose, insulin, an immune system humoral factor, or the like permeating the immunoisolation membrane can be measured by encapsulating a sample solution of a known concentration of insulin or the like dissolved in PBS in an embedding chamber covered with the immunoisolation membrane, immersing it in a certain amount of PBS, and quantifying the amount of each substance eluted out of the immunoisolation membrane after a certain period of time at 37°C by ELISA.

In the present specification, the permeability of glucose, insulin, an immune system humoral factor, or the like through the immunoisolation membrane is a value expressed as a percentage of what the permeation amount of each substance after 24 hours, measured by the above method, is with respect to the amount of the substance initially encapsulated in the embedding chamber covered with the immunoisolation membrane, unless otherwise specified.

The permeabilities of insulin and glucose through the immunoisolation membrane of the present invention after 24 hours are preferably 90% or more, and more preferably 95% or more.

The permeability of immune system humoral factors through the immunoisolation membrane of the present invention after 24 hours is preferably 30% or less, and more preferably 10% or less. In the present invention, it is preferred that the immunoisolation membrane is capable of removing 50% or more of immunoresponsive cells and immune system humoral factors, and it is more preferred that the immunoisolation membrane is capable of removing 90% or more of immunoresponsive cells and immune system humoral factors. The percentage of immunoresponsive cells removed can be measured by a cell invasion test using a Boyden chamber method, in which an immunoisolation membrane is attached to insert wells, and cell migration is measured. The percentage of immune system humoral factors removed can be measured by a permeability test, in which a solution containing immune system humoral factors is filtered or diffused through an immunoisolation membrane.

The hydrogel for embedding of the present invention can prevent immunoresponsive cells from attacking the material to be transplanted even when the immunoresponsive cells pass through the immunoisolation membrane and enter the embedding chamber. One embodiment of the present invention is described below with reference to the drawings.

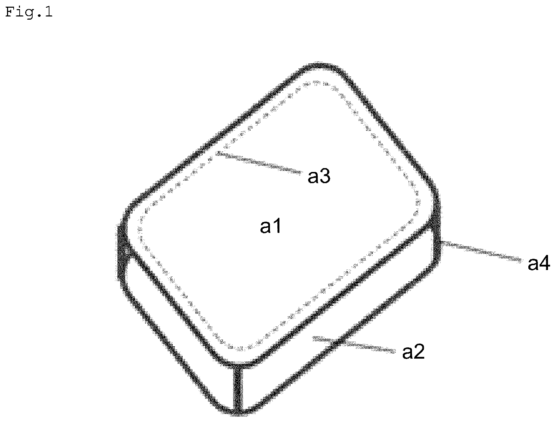

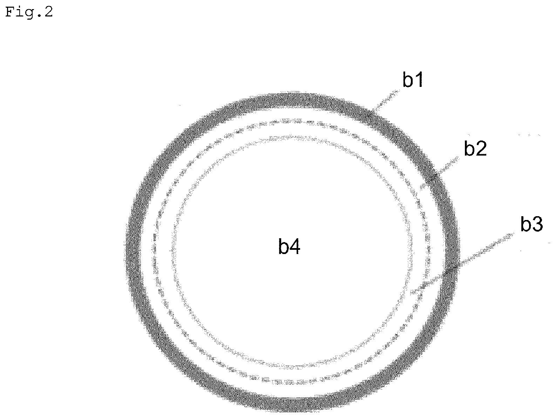

As schematic views of the macroencapsulation immunoisolation device in which a hydrogel for embedding is encapsulated in an embedding chamber surrounded by an immunoisolation membrane, Fig. 1 shows an immunoisolation membrane molded into a bag, and Fig. 2 shows an immunoisolation membrane molded into a tube. The bag-shaped device (Fig. 1) is molded by fusing (a3) immunoisolation layers (a1 and a2) comprising multiple layers as shown below by using heat, ultrasound, high frequency, or electron beam while having a certain distance (a4) to ensure a space for embedding a cell fixation layer (embedding chamber). A spacer may be provided to ensure a certain distance (a4).

The tubular device (Fig. 2) is formed of immunoisolation layers (b1, b2, and b3) molded into a tubular shape. The tubular device is molded by embedding the immunoisolation membrane in the tubular interior (b4), and fusing and sealing both of the tubular ends by heat, ultrasound, high frequency, electron beam, or the like.

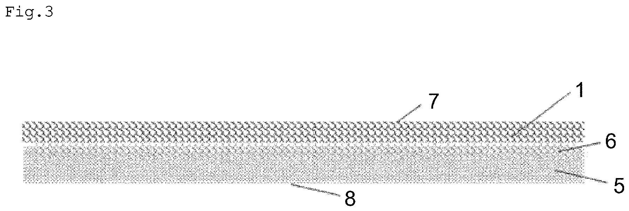

Fig. 3 shows one example of a concept view of the isolation layer of the device. Multiple layer formation is performed using the porous membrane (1) and the hydrogel layer (5) as an outer layer and an inner layer, respectively, via a joining part (6). The outermost layer (7) is in contact with the transplantation site of a host side, and the innermost layer (8) is in contact with the immunoisolation membrane.

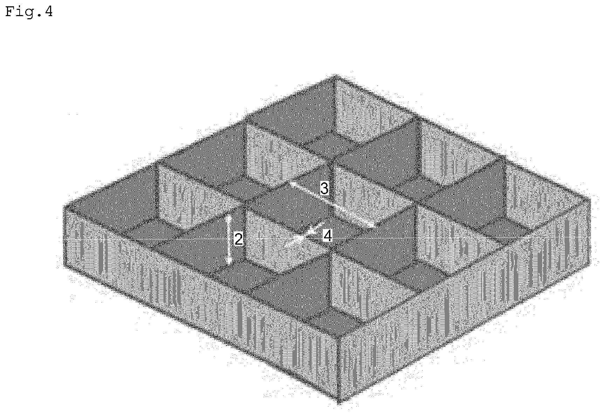

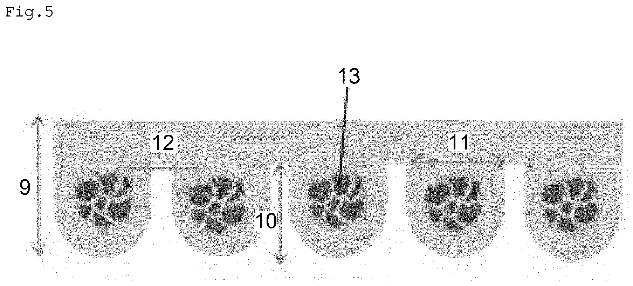

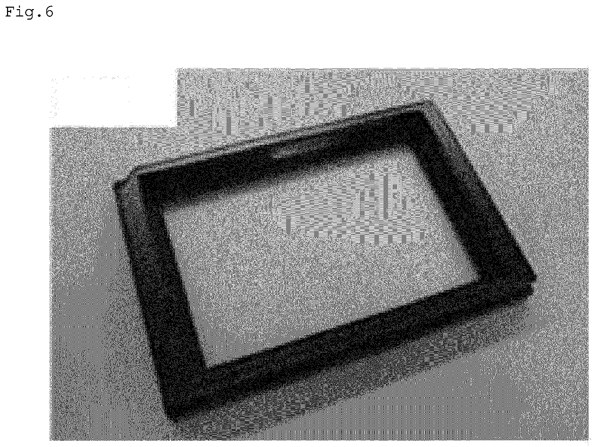

Fig. 4 shows an example of a mold for producing a hydrogel for an immunoisolation membrane using a microfabrication technique, for dispersing and fixing cells and the like in a substantially uniform manner. The mold comprises microdimples having a certain height (2) and width (3), and walls (4) for partition. Fig. 6 shows an entire view of the mold. Fig. 5 shows a concept view of the hydrogel sheet comprising cells and the like, which is produced using the mold. In Fig. 5, 9 indicates the height of the entire device, 10 indicates the height of the cell-embedded part, 11 indicates the width of the cell-embedded part, 12 indicates the distance between the cell-embedded parts, and 13 indicates the embedded cells.

The present invention relates to a device for transplantation for use in cell transplantation therapy and to a transfection material. In particular, the present invention relates to an immunoisolation device for protection against immune rejection reaction of a material to be transplanted due to transplantation, and a transplantation material.

As shown in Fig. 1 or Fig. 2, the device concept view shows that the device has a bag or tubular shape. In the device, a material to be transplanted, which is to be transplanted, such as cells or cell clusters, is embedded inside the space surrounded by an immunoisolation membrane. The immunoisolation membrane indicates a membrane in which the material to be transplanted, such as cells or cell clusters, is fixed in a dispersed state in a substantially uniform manner in a hydrogel inside an embedding chamber.

The immunoisolation membrane has a function of uniformly fixing cells by adjusting the crosslinking strength and/or gel strength of a fixation material such as a hydrogel, and can also have a role of preventing immune system humoral factors such as antibodies from permeating the graft. In the hydrogel for embedding a material to be transplanted, which is used as a cell fixation material, it is possible to suppress permeation of immune system humoral factors by adjusting the gel crosslinking degree and/or gel strength, without suppressing the release of physiologically active substances from cells and the like. Accordingly, the hydrogel for embedding a material to be transplanted according to the present invention may be used as a transplantation material by embedding a material to be transplanted without using an immunoisolation membrane in combination to form a transplantation material. Alternatively, a material to be transplanted may be embedded in a hydrogel, and further covered with an immunoisolation membrane to form a transplantation material.

In order to maintain the viability and functionality of the material to be transplanted such as cells after transplantation, adjusting the gel strength etc.; and uniformly dispersing and fixing the material to be transplanted such as cells and the like embedded in a gel without aggregation or localization are important. Additionally, maintaining a constant spacing between cells and the like and keeping the constant thickness of the gel layer are also important to supply oxygen and/or nutrients to the cells and the like.

Although it is possible to disperse and fix cells and the like by suspending and dispersing the cells and the like in a hydrosol solution, and by gelling the resultant into a sheet, suppressing the aggregation of cells and the like to provide a certain distance between the cells and the like is not easy. Accordingly, a plastic sheet containing many microdimples having a diameter of 50 to 500 µm and a depth of 100 to 500 µm on the bottom surface of the sheet, and having a space between individual microdimples of 50 µm or less is produced by microfabrication technology etc., and using this sheet as a mold, a hydrogel sheet is produced. This enables efficient and uniform dispersion and fixation of cells and the like with a certain space between the cells and the like, without aggregation or localization of the cells and the like.

In order to fully disperse oxygen and/or nutrients for inner cells by providing a certain space between the cells and the like, and suppressing the aggregation of cells and the like, it is desirable that a space of 10 µm or more is maintained. In order to reduce the volume of the embedding chamber while encapsulating the number of cells required for attaining a therapeutic effect, thereby reducing the size of the transplantation material, it is desirable that the space between the cells is 500 µm or less.

The gelation can be easily achieved by photocrosslinking by irradiation with visible light in a visible light range of 360 nm or more (e.g., 365 nm) for at least one minute, which does not adversely affect the cells and the like.

In place of a hydrogel sheet, a microcapsule bead having a diameter of 500 µm or less, in which cells and the like suspended and dispersed in the same hydrogel solution are microencapsulated by a microreactor method or the like, and then gelation is performed by photocrosslinking, can be used.

### Examples

The invention is described in more detail below on the basis of examples; however, it goes without saying that the invention is not limited to these Examples.

### Example 1 (Gel production and control of functionality by gel concentration)

### 1) Synthesis of photocurable vinyl alcohol-based polymer

40 g of PVA117 (polyvinyl alcohol (trade name "PVA117," saponified product of polyvinyl acetate, average polymerization degree of 1700, saponification degree of about 98.0 to 99.0 mol%, viscosity (4%, 20°C) of 25.0 to 31.0 mPa·s, produced by Kuraray Co., Ltd.) was used, and dissolved in 350 mL of dimethyl sulfoxide (DMSO) at 80°C for 4 hours; and then 2.1 g (18.7 mmol) of vinyl methacrylate was added thereto. The mixture was further stirred at 80°C for 3 hours. After cooling, a reaction solution was poured into 2 L of methanol with stirring. Stirring was stopped and the mixture was allowed to stand for 1 hour. The obtained solids were collected, and further immersed in 1 L of methanol for 1 hour, followed by washing. This washing operation was performed a total of three times. The collected solids were vacuum-dried overnight at room temperature to give methacryloylated PVA117. The methacryloylated PVA117 had an introduction rate of an ethylenically unsaturated group (methacryloyloxy group) of 2.0 mol% relative to the repeating units of raw material PVA (hereinafter abbreviated as "MA-PVA117 (2.0)") .

88 mL of ion-exchanged water was added to 12 g of MA-PVA117 (2.0), and dissolving was performed under stirring at 80°C for 4 hours. After the resultant was cooled to room temperature, L0290 (lithium salt of phenyl(2,4,6-trimethylbenzoyl)phosphinate (photoradical polymerization initiator, trade name "L0290," produced by Tokyo Chemical Industry Co.), which ws a watersoluble photoradical polymerization initiator, was added to this MA-PVA117 (2.0) aqueous solution to a concentration of 0.1 mass%, and dissolved, thus preparing an uncured gel solution (hereinafter abbreviated as "photocurable PVA117").

In a similar procedure, using PVA124 (polyvinyl alcohol (trade name "PVA124," saponified product of polyvinyl acetate, average polymerization degree of 2400, saponification degree of about 98.0 to 99.0 mol%, viscosity (4%, 20°C) of 40.0 to 48.0 mPa·s, produced by Kuraray Co., Ltd.)) and PVA235 (polyvinyl alcohol (trade name "PVA235," average polymerization degree of 3500, saponification degree of about 87.0 to 89.0 mol%, viscosity (4%, 20°C) of 45.0 to 52.0 mPa.s, produced by Kuraray Co., Ltd.)), uncured gel solutions (hereinafter referred to as "photocurable PVA124" and "photocurable PVA235") having different polymerization degrees were prepared.

### 2) Production of PVA hydrogel and evaluation of substance permeability

Each of 0.1 ml of hydrosols in which the photocurable PVA obtained by the above synthesis procedure was adjusted to a concentration of 8 mass% or 4 mass% in PBS was injected into a mold container of W 16 mm × D 12.5 mm × H 0.5 mm, and irradiation was performed with a visible light of 365 nm for 3 minutes to prepare hydrogels of sample A (photocurable PVA117, concentration: 8 mass%), sample B (photocurable PVA117, concentration: 4 mass%, sample C (photocurable PVA124, concentration: 4 mass%), and sample D (photocurable PVA235, concentration: 4 mass%).

Subsequently, the protein permeability test was performed using the samples A, B, C, and D. PVA hydrosol solutions in which insulin (30 U/L), glucose (5 mg/mL), and IgG (0.5 pg/mL) were adjusted were photocured by photocrosslinking, and these samples were immersed in 20 mL of PBS solution to collect 0.5 mL of PBS solution over time, thus measuring the amount of each substance in the PBS solution by ELISA.

As shown in Table 1, the results confirm the following.

In all of the samples A, B, C, and D, the glucose permeability after 24 hours was 100%.

In all of the samples A, B, C, and D, the insulin permeability after 24 hours was 95% or more.

In all of the samples A, B, C, and D, the IgG permeability after 24 hours was 10% or less.

As compared to the sample A having a gel concentration of 8 mass%, the permeabilities of the samples B, C, and D having a gel concentration of 4 mass% were high.

**Table 1**

| | Sample A | | Sample B | | Sample C | | Sample D | |
|---|---|---|---|---|---|---|---|---|
| Measurement time | 30 min. | 24 hr. | 30 min. | 24 hr | 30 min. | 24 hr | 30 min. | 24 hr |
| Glucose | 100% | 100% | 100% | 100% | 100% | 100% | 100% | 100% |
| Insulin | 68% | 95% | 92% | 100% | 83% | 100% | 91% | 100% |
| IgG | 0.9% | 1.6% | 1.7% | 2.8% | 1.4% | 2.3% | 1.7% | 2.7% |

### 3) Functionality evaluation (embedding and fixation of cells)

The sample A solution containing mouse fibroblast NIH/3T3 cells was placed in a 24-well multi-well plate, and irradiated with a visible light of 365 nm for 3 minutes to form a hydrogel. Further, culturing was performed at 37°C and 5% CO₂, using a Dulbecco's modified Eagle's medium (DMEM) (Sigma) containing 15% fetal bovine serum, 100 U/ml penicillin, and 100 µl/ml streptomycin. 24 hours later, the cells were removed, and the state of the cells was observed using a phase-contrast microscope. The cells maintained their spherical shape, which allowed for the embedding and fixation of NIH/3T3 cells in a PVA hydrogel.

A device in which mouse fibroblast NIH/3T3 cells (2*10⁶ cells) were encapsulated in the hydrogel was prepared, and cultured in the wells of a 6-well plate. The culture surface was observed on day 7, and cells attached to the wells and a culture supernatant were collected to count cell nuclei. The results indicate that no cell invasion was observed in all of the samples A, B, and C.

Rat pancreatic islet cells (number of pancreatic islets: 1,000) were suspended in each of the hydrosol solutions in which photocurable PVA117 was adjusted to a concentration of 8 mass% in a Dulbecco's modified Eagle's medium (DMEM) (Sigma Corporation) containing 15% fetal bovine serum, 100 U/ml penicillin, and 100 µl/ml streptomycin in a sterile environment according to the sample trial production conditions of Section 2) above. The resultant was injected into a mold container (0.1 ml) of W 16 mm × D 12.5 mm × H 0.5 mm, followed by irradiation with a visible light of 365 nm for 3 minutes, thus producing a hydrogel embedding pancreatic islet cells.

Subsequently, the hydrogel was intraperitoneally implanted (n=4) into STZ diabetic model mice to perform a blood glucose lowering test. As a result, in the hydrogel transplantation group, blood glucose levels varied from 440-520 mg/dl to 180-240 mg/dl and remained normoglycemic during the one-week period beginning the day after transplantation..

### Example 2 (Microdimpled sheet)

Mouse pancreatic β-cell tumor-forming cell line MIN6 cells were seeded into a floating cell culture flask (Sumitomo Bakelite Co., Ltd.), and cultured for one week to obtain 2000 or more cell aggregates having a diameter of about 150 µm. About 2300 MIN6 cell aggregates obtained above were suspended in the sample C (4% MA-PVA124 PVA solution, 1 mL) as in Example 1, and 250 µL of the PVA solution containing the above cell aggregates was inject into a 24-hole container with an SBS standard size (W 128 mm × D 85 mm × H 20 mm) containing a microdimple sheet having a diameter of 500 pm, a height of 400 µm, and a pore interval of 30 pm, followed by irradiation with a visible light of 365 nm for 3 minute, thus producing a hydrogel sheet having a thickness of 500 µm.

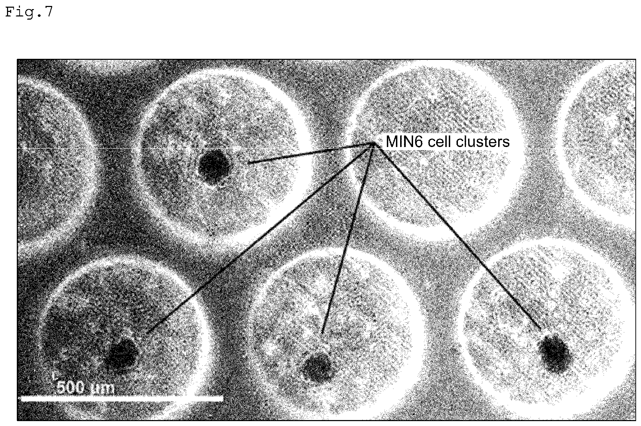

The obtained hydrogel sheet was immersed in PBS, and observed using an inverted microscope; and the following was confirmed. Gel-embedding was performed in the state in which cell aggregates were in microdimples. This allows for the production of the hydrogel sheet in which the cells were placed with appropriate spaces (Fig. 7).

### Description of Reference Symbols

a1 Immunoisolation layer
a2 Immunoisolation layer
a3 Fusing
a4 Certain distance
b1 Immunoisolation layer
b2 Immunoisolation layer
b3 Immunoisolation layer
b4 Tubular interior
1 Porous membrane
2 Height
3 Width
4 Thickness
5 Hydrogel layer
6 Joining part
7 Outermost layer
8 Innermost layer
9 Height of entire device
10 Height of cell-embedded part
11 Width of cell-embedded part
12 Distance between cell-embedded parts
13 Embedded cell

## Claims

1. A hydrogel for embedding a material to be transplanted, comprising a polymer crosslinked by visible light, and water.

2. The hydrogel for embedding a material to be transplanted according to claim 1, wherein the polymer is a polyvinyl alcohol-based polymer.

3. An immunoisolation device comprising the hydrogel for embedding a material to be transplanted according to claim 1 or 2.

4. A transplantation material comprising a material to be transplanted that is embedded in the hydrogel according to claim 1 or 2.

5. A transplantation material comprising a material to be transplanted that is embedded in a hydrogel in an embedding chamber covered with an immunoisolation membrane of a macroencapsulation immunoisolation device, the hydrogel comprising a polymer crosslinked by visible light, and water.

6. The transplantation material according to claim 5, wherein the immunoisolation membrane comprises a hydrogel.

7. The transplantation material according to claim 5 or 6, wherein the visible light has a wavelength of 360 nm or more.

8. The transplantation material according to any one of claims 5 to 7, wherein the immunoisolation membrane is capable of removing 50% or more of immunoresponsive cells and immune system humoral factors.

9. The transplantation material according to any one of claims 5 to 8, wherein the immunoisolation membrane has insulin and glucose permeabilities of 95% or more and an immune system humoral factor permeability of 10% or less, without permeation of immunoresponsive cells.

10. The transplantation material according to any one of claims 5 to 9, wherein the immunoisolation membrane has a thickness of 20 µm or more and 500 µm or less, the material to be transplanted is a plurality of cells or cell clusters, and the plurality of cells or cell clusters embedded in the hydrogel are arranged at an interval of 10 µm or more and 500 µm or less without contacting each other.
